# DEMANDE DE BREVET EUROPEEN

(11) **EP 0 821 922 A1**
(43) Date de publication de la demande: **04.02.1998**
(21) Numéro de dépôt: 97460029.8
(22) Date de dépôt: 28.07.1997
(51) Int. Cl.: A61F 2/32, A61F 2/30, A61F 2/46

(54) **Partie articulaire d'une prothèse de hanche avec piége à particules**

(30) Priorité: 29.07.1996 FR 9609768
(71) Demandeur: Hubin, Claude, 17260 Gémozac (FR); Sterpin, Marie-Jean, 17260 Gémozac (FR)
(72) Inventeur: Hubin, Claude, 17260 Gémozac (FR)
(74) Mandataire: Schmit, Christian Norbert Marie

(57) **Abrégé**

Dans la partie articulaire d'une prothèse de hanche selon l'invention comportant une tête (1) avec une surface de glissement convexe de forme sphérique (10) destinée à coopérer avec une surface de glissement concave (40) de même forme sphérique et de même diamètre d'une pièce de réception de la tête (4), le corps de la tête (1) et celui de la pièce (4) sont métalliques. D'autre part, il est prévu un piège à particules (42) sous la forme d'une fossette ménagée dans ladite surface de glissement concave (40) de la pièce (4) de réception de la tête (1). Les surfaces de glissement sont réalisées en métal dur "hyperlisse" ou sous forme de revêtement en céramique.

## Description

La présente invention concerne les prothèses de hanche, et plus particulièrement leur partie articulaire.

La totalité des prothèses de hanche comprennent un élément de remplacement de la partie supérieure du fémur dit implant fémoral, qui consiste en une tête à surface de glissement de forme sphérique raccordée par un col à une tige destinée à être ancrée dans la partie restante du fémur.

La tête de l'implant fémoral s'articule dans la cavité cotyloïdienne. Si le cartilage dans celle-ci est en bon état, la tête de la prothèse coopère avec la surface de glissement naturelle, de préférence par l'intermédiaire d'une cupule mobile. Si au contraire le cartilage est déficient, il est nécessaire de prévoir un implant cotyloïden, qui constitue avec l'implant fémoral un couple dénommé prothèse de hanche totale.

L'invention concerne l'ensemble des prothèses dont la partie articulaire comporte au moins deux éléments prothétiques mobiles l'un par rapport à l'autre, soit donc un couple tête fémorale-cupule mobile, ou un couple tête fémorale-cotyle, ou encore un arrangement avec un élément supplémentaire : tête fémorale-pièce intermédiaire mobile-cotyle.

L'invention a été réalisée dans le but d'améliorer globalement cette partie essentielle des prothèses de hanche, pour en augmenter la fiabilité et la durée de vie. Il s'agissait, autrement dit, de rechercher des solutions répondant au mieux et le plus complètement possible aux multiples problèmes que l'on rencontre actuellement : notamment en augmentant la qualité du glissement, supprimant le risque de fracture des pièces en présence, limitant leur usure, limitant les risques de descellement.

Dans sa forme générale, l'invention est une partie articulaire de prothèse de hanche comportant une tête de remplacement de la tête fémorale avec une surface de glissement convexe de forme sphérique destinée à coopérer avec une surface de glissement concave de même forme sphérique et de même diamètre d'une pièce de réception de la tête, dans laquelle le corps de ladite pièce et celui de la tête sont métalliques, et caractérisée d'autre part en ce qu'elle comprend un piège à particules sous la forme d'une fossette ménagée dans ladite surface de glissement concave de ladite pièce de réception de la tête.

Dans cette solution selon l'invention, l'utilisation de polyéthylène ou équivalent, malgré ses qualités de glissement reconnues et la prétendue souplesse qu'il peut apporter, a été écartée pour deux raisons qui sont liées et que sont la médiocrité de sa résistance à l'usure et, plus grave, le fait avéré que la formation de débris d'usure de polyéthylène entraîne le développement de fibrose qui s'introduit dans les interfaces os-prothèse, en détruisant l'os avec risque de descellement à moyen terme, tout spécialement dans le cas des prothèses cimentées.

Ont également été écartées les pièces à corps en céramique, qui ont comme avantage de réunir d'excellentes qualités de glissement à une relative facilité de fabrication, mais dont le risque de fracture est important.

Selon l'invention, les surfaces de glissement des diverses pièces sont des surfaces sphériques de corps métalliques. En pratique, elle peuvent consister en une paroi en métal dur hyperlisse réalisée de manière connue en soi sous forme de revêtement ou de traitement de surface du métal constituant la pièce, ou en une couche en céramique rapportée sur le métal. La couche en céramique peut être réalisée par une technique de dépôt sous vide, avec des composants biocompatibles : une vapeur métallique fortement ionisée réagit avec un gaz également ionisé pour conduire à la synthèse d'un composé (tel que TiN, TiCN, CrN, etc.) qui se dépose en une couche fine de 2 à 10 µm, environ, sur un substrat auquel elle adhère fortement. L'opération peut être répétée pour obtenir un revêtement multicouche.

Le piège à particules selon l'invention a pour fonction première de préserver les surfaces de glissement en contact : plutôt que de rester des éléments d'abrasion actifs entre ces surfaces, les particules qui pour diverses raisons ont pu s'interposer profondément entre elles finissent toujours assez rapidement par atteindre la zone dudit piège dont elles deviennent alors prisonnières.

Le piège à particules a d'autre part pour avantage d'améliorer le glissement par diminution de la zone de contact de deux surfaces de glissement coopérant ensemble. Concrètement, il permet d'utiliser des têtes fémorales de diamètre augmenté tout en conservant une surface de contact de grandeur inchangée avec une pièce de réception pareillement enveloppante.

De préférence, le piège à particules contient le pôle de la surface de la pièce dans laquelle il est ménagé. Sur la tête fémorale, on entend ici par pôle la région de sa surface externe opposée à sa zone de raccordement avec le col de prothèse ; et les pôles des autres pièces de l'articulation fémorale sont les régions de celles-ci qui correspondent au pôle de la tête en position neutre de l'articulation.

On peut tirer profit de la position polaire du piège à particules pour utiliser une autre technique avantageuse de formation d'un revêtement en céramique sur une pièce métallique, dite technique de la "céramique projetée". Il s'agit d'un procédé bien connu pour réaliser des portées tournantes de pièces mécanique de grande résistance, sur des arbres ou dans des paliers par exemple. Une épaisseur de 0,25 à 0,3 mm, environ, de céramique est projetée par un plasma d'arc soufflé sur toute la surface considérée de la pièce pendant que celle-ci tourne autour d'un axe. La projection se fait de préférence entre deux rebords métalliques, ce qui permet sa mise en compression sur couche d'accrochage. Les céramiques utilisées sont formées à partir de Al₂O₃, Cr₂O₃, ZrO₂, TiN, etc. Dans le cadre de la présente invention, d'autres substances pourront être utilisées, pour autant que le type de céramique soit choisi essentiellement d'après sa dureté, sa résistance à l'usure, ses qualités de frottement et sa biocompatibilité.

On comprend que les revêtements réalisables avec cette technique ne peuvent avoir leur continuité assurée au voisinage de l'axe autour duquel s'effectue la rotation rapide indispensable des pièces pendant la projection. Dans l'application aux pièces d'articulation fémorale, la projection de la céramique serait perturbée aux pôles, et l'artifice d'utiliser un excentrique ne ferait qu'étendre la zone de fragilité. Par conséquent, prévoir des cavités dans ces zones des surfaces de glissement pour y éliminer le contact avec les surfaces coopérant avec elles est une façon simple et efficace de remédier au problème. Bien entendu, la tête fémorale sera alors elle aussi prévue avec un aplatissement ou méplat, ou de préférence une cavité polaire.

Les caractéristiques et avantages de l'invention mentionnés ci-dessus, ainsi que d'autres, apparaîtront plus clairement dans la description qui suit, faite en relation avec les dessins joints, dans lesquels :
la Fig. 1 est une vue schématique en coupe d'une partie articulaire selon l'invention dans une prothèse simple, c'est-à-dire constituée d'un seul implant fémoral, sans cotyle prothétique, mais plus exactement dite prothèse intermédiaire du fait de comporter une cupule mobile sur la tête fémorale ;
la Fig. 2 est une vue schématique en coupe de la partie articulaire d'une prothèse de hanche totale, comprenant donc un couple tête fémorale-cotyle prothétique ;
la Fig. 3 est une vue schématique en coupe de la partie articulaire d'une autre prothèse de hanche totale comprenant une pièce supplémentaire dite pièce intermédiaire faisant interface entre tête et cotyle ;
la Fig. 4 est une vue en plan du cotyle prothétique représenté à la Fig. 3 ; et
la Fig. 5 est une vue en plan de la pièce supplémentaire également représentée à la Fig. 3.

Dans l'ensemble des dessins, les mêmes références ont été utilisées pour désigner partout les mêmes éléments.

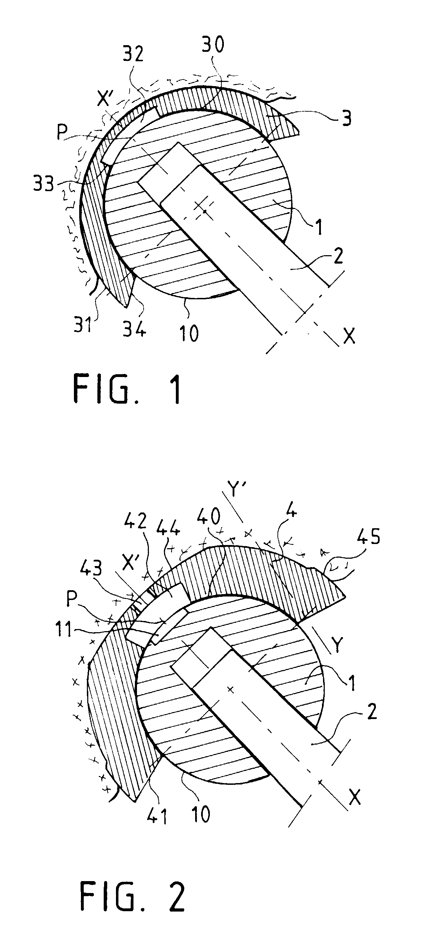

Les Figs. 1 à 3 illustrent donc à titre non limitatif l'invention dans trois types d'articulation de prothèse de hanche qui sont représentatifs des principales solutions existantes à l'heure actuelle en fonction des besoins.

Considérant d'abord la version de la Fig. 1 qui est la plus simple puisque conçue pour coopérer avec une cavité cotyloïdienne fonctionnellement conservée, elle comporte un couple formé d'une tête fémorale 1 et d'une cupule mobile 3, grâce auquel le glissement articulaire est dédoublé entre, d'une part, les surfaces coopérantes 10 et 30 respectivement de la tête et la cupule, et d'autre part, la surface externe 31 de la cupule et la surface de glissement naturelle de la cavité cotyloïdienne, en vue d'éviter d'y solliciter excessivement le cartilage.

La tête 1 et la cupule 3 sont des pièces à corps métallique. La tête 1 est représentée emmanchée selon un système de cône morse classique sur le col 2 d'une tige fémorale, non représentée, sachant qu'en variante, la tête pourrait tout aussi bien ne constituer qu'une seule pièce avec la tige.

La cupule 3, de forme globalement hémisphérique, enveloppe légèrement plus que la moitié hémisphérique de la tête 1 pour éviter les risques de déboîtement. L'emboîtement doit pouvoir se faire en force durant l'intervention chirurgicale, grâce à un ajustage précis. La surface externe de forme sphérique 31 de la cupule 3, destinée à coopérer avec le cotyle naturel, peut être légèrement augmentée par relèvement vers l'extérieur de son bord équatorial 34, comme montré sur le dessin.

La surface interne 30 de la cupule 3, de même forme et de même rayon que la tête 1, a son centre décalé sur l'axe polaire de la cupule (ici confondu avec l'axe XX' de la tête et du col) par rapport à celui de la surface externe 31, de telle manière que l'épaisseur de la cupule décroisse de l'équateur jusqu'au pôle, et qu'ainsi en situation de compression normale entre le cotyle et la tête, elle soit rappelée constamment et automatiquement en position correcte.

Conformément à l'invention, la surface de glissement interne 30 de la cupule 3 présente une zone en creux ou cavité 32 relativement centrale et qui, de préférence, contient son pôle P. Dans l'exemple représenté, il s'agit d'une cavité circulaire 32 centrée sur le pôle P de la surface 30, et dont le bord périphérique 33 est sensiblement radial par rapport à la surface 30. Tout en ayant un effet bénéfique au glissement de diminution en superficie du contact entre les surfaces de glissement 10 et 30 pour un diamètre et un enveloppement de la tête donnés, la cavité 32 a pour fonction de piéger les particules d'usure ou autres qui peuvent se trouver interposées profondément entre les surfaces 10 et 30.

Chacune de surfaces de glissement 10, 30 et 31 peut être une paroi en métal dur "hyperlisse", réalisée par revêtement ou traitement de surface des pièces 1 et 3, lesquelles sont forgées par exemple dans un alliage tel que chrome-cobalt-molybdène. En variante, chacune des surfaces 10, 30 et 31 peut être un revêtement en céramique. Comme explicité précédemment, dans le cas où il est fait appel à la technique de la "céramique projetée" pour réaliser une de ces surfaces, et pour en faciliter la mise en oeuvre, ladite surface est avantageusement prévue avec une discontinuité de forme dans sa région polaire, par laquelle est supprimé tout contact fonctionnel de glissement de cette région. Pour la surface 30, cette discontinuité consiste en la cavité 32, laquelle est alors avantageusement circonscrite par un étroit rebord métallique d'arrêt du revêtement en céramique, lequel peut être limité équatorialement de la même façon. Pour la surface 10 de la tête, ladite discontinuité de forme peut être un simple aplatissement polaire. S'il s'agit d'une cavité, elle est bien entendu elle aussi de préférence circonscrite par un rebord métallique d'arrêt de la céramique.

La Fig. 2 représente la partie articulaire d'une prothèse de hanche totale selon l'invention, avec son cotyle 4 dont le corps est métallique en position fonctionnelle dans l'os. L'ensemble de la tête 1 et du col 2 de l'implant fémoral est le même qu'à la Fig. 1, sauf qu'ici, la tête 1 présente dans sa surface de glissement une fossette ou cavité polaire 11.

Classiquement, le cotyle 4 a extérieurement la forme d'une demi-sphère, présentant un aplatissement ou méplat polaire 44 et un léger épaississement équatorial 45, utiles pour la stabilité immédiate obtenue par impaction dans la cavité de l'os (effet "press-fit"). La surface extérieure du cotyle 4 comporte avantageusement un revêtement métallique réhabitable, qui peut lui-même être recouvert d'hydroxyapatite.

Intérieurement, le cotyle 4 présente une surface de glissement hémisphérique 40 destinée à recevoir la tête 1 et délimitée par un bord équatorial 41. De préférence, le bord équatorial 41 est incliné montant de l'intérieur vers l'extérieur (dans le cotyle vu avec son axe polaire vertical et sa surface 40 orientée vers le haut) pour réduire le risque de luxation. Le bord équatorial 41 doit d'autre part avoir une surface dure pour éviter la formation de débris métalliques en cas de luxation. En pratique, il pourra être traité ou revêtu de la même façon que la surface de glissement 40 à laquelle il est adjacent.

Conformément à l'invention, la surface de glissement 40 comporte un piège à particules 42 contenant son pôle P, s'agissant ici plus précisément d'une cavité centrée sur le pôle P et présentant un bord sensiblement radial (perpendiculaire à la surface 40). La cavité 42 est prolongée par un alésage polaire taraudé 43 axé radialement, soit ici selon XX', qui permet de fixer solidement un impacteur sur le cotyle. De plus, l'alésage 43 est traversant, de sorte que l'on peut s'assurer visuellement et par palpation après impaction qu'un contact satisfaisant a été obtenu entre le cotyle et l'os.

Chacune des surfaces de glissement 10, 40, et de même celle du bord équatorial 41, peut être en métal dur hyperlisse ou en céramique. Elle peut être réalisée de la même façon et avoir les mêmes caractéristiques que les surfaces de glissement 10, 30 et 31 de l'exemple précédemment décrit.

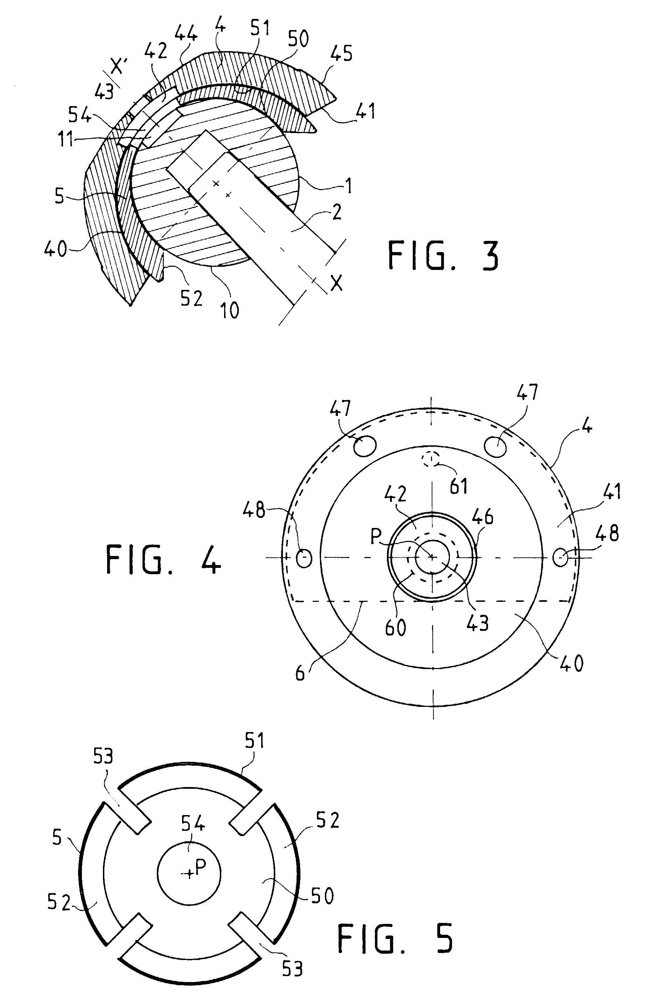

L'ensemble articulaire de la Fig. 3 diffère de celui de la Fig. 2 en ce qu'il comprend une pièce supplémentaire, à savoir une pièce intermédiaire mobile 5 qui s'interpose entre la tête 1 et le cotyle 4, ayant pour fonction de dédoubler le glissement entre, d'une part, sa surface interne 50 et la surface externe 10 de la tête 1 et, d'autre part, sa surface externe 51 et la surface de glissement 40 du cotyle 4.

La partie supérieure de l'implant fémoral regroupant la tête 1 et le col 2 est la même qu'à la Fig. 2, excepté que la tête 1 a un diamètre moindre. Le cotyle 4 regroupe également les mêmes caractéristiques que celui de la Fig. 2 et ne sera donc pas redécrit maintenant.

La pièce 5 a un corps métallique, et comme la cupule 3 de la Fig. 1, elle enveloppe la tête 1 sur légèrement plus que sa moitié hémisphérique. Elle est emboîtée sur la tête 5 en fin du cycle de fabrication, en ayant recours à des artifices thermiques, ou bien en per-opératoire, et comme le montre la Fig. 5, elle comporte avantageusement à cet effet une pluralité de fentes 53 à partir de son bord équatorial 52, dirigées vers son pôle P, qui lui confèrent de la souplesse. Elle comporte une surface de glissement interne 50 et une surface de glissement externe 51, toutes deux de forme sphérique, et respectivement de mêmes rayons que la tête 1 et la surface de glissement 40 du cotyle 4. Comme dans la cupule 3, les surfaces interne et externe 50 et 51 ont des centres décalés sur l'axe polaire, afin que l'épaisseur de la pièce 5 décroisse progressivement de son bord équatorial jusqu'en son pôle, et qu'il se crée ainsi un rappel automatique en position correcte de la pièce 5, de par la compression permanente qui s'exerce naturellement sur l'articulation. Comme dans la cupule 3 également, le bord équatorial 52 est de préférence relevé vers l'extérieur pour augmenter encore la surface de glissement externe 51.

Conformément à l'invention, la pièce intermédiaire mobile 5 comporte elle aussi son piège à particules 54 dans sa surface 50. De préférence, il s'agit comme représenté sur le dessin d'un trou traversant axé sur son pôle P et qui va se trouver ainsi en correspondance, et donc fonctionnellement en communication, avec la cavité 42 du cotyle 4.

Comme dans les exemples précédents, chacune des surfaces de glissement 10, 40, 50, 51, de même que la surface du bord équatorial 41 du cotyle, peut être en métal dur "hyperlisse" ou en céramique, et réalisée selon l'une des techniques mentionnées.

La Fig. 4 est une vue en plan du cotyle 4 de la Fig. 3, qui illustre des caractéristiques qu'il peut avoir en commun avec le cotyle de la Fig. 2. Sa cavité polaire 42 est montrée entourée d'une bordure métallique étroite 46 d'arrêt pour un revêtement en céramique constituant la surface de glissement 40.

Le bord équatorial 41 présente deux trous 47 qui débouchent dans la surface externe du cotyle, et qui sont destinés à recevoir des vis de fixation dans l'os iliaque, schématiquement matérialisés par l'axe YY' à la Fig. 2. Deux autres trous 48, diamétralement opposés, sont situés de part et d'autre des trous 47. Les trous 48 sont utilisables pour l'extraction du cotyle et sont taraudés à cet effet. Mais ils servent principalement au positionnement d'une instrumentation de pose qui comporte essentiellement un bloc 6 représenté schématiquement en traits interrompus à la Fig. 4. Le bloc 6 vient s'engager sur le bord équatorial 41 du cotyle 4 par sa face de dessous dont deux tétons pénètrent dans les trous 48. Le bloc 6 est immobilisé et fixé sur le cotyle au moyen d'une tige d'impaction qui le traverse par son trou 60 et vient se visser dans l'alésage polaire 43 au fond de la cavité 42. L'impaction en force se fait donc en prenant appui sur l'équateur du cotyle, la surface de glissement étant totalement préservée.

Le repère 61 désigne un trou taraudé débouchant dans la surface supérieure du bloc 6, qui est destiné à recevoir une tige de repérage. Cette tige matérialise la verticale lorsque le cotyle est en place dans l'acétabulum, le patient étant en décubitus latéral.

Comme pour les prothèses existantes, il existera une gamme dimensionnelle appropriée des diverses pièces d'articulation selon l'invention. Le cotyle aura par exemple un diamètre extérieur qui augmentera par pas de 2 mm à partir d'une valeur minimale de 46 mm.

S'agissant de la tête, le fait que son corps soit métallique permettra une grande variablité au niveau de son évidement de raccordement à un col d'implant fémoral, pour un ajustement fin de la longueur de col. D'autre part, étant donné la diminution de surperficie de contact entre surfaces de glissement résultant de la présence des pièges à particules, une même qualité de glissement (fonction de la superficie de contact) sera obtenue avec une tête de diamètre augmenté par rapport à celui d'une tête classique, alors qu'il est bien connu que l'augmentation du diamètre réduit le risque de luxation. En pratique, le diamètre intermédiaire normalisé de 28 mm devrait être un compromis satisfaisant, qui offrira la même qualité de glissement que le diamètre de 22,2 mm dans les articulations actuelles.

## Revendications

1. Partie articulaire d'une prothèse de hanche, comportant une tête (1) de remplacement de la tête fémorale avec une surface de glissement convexe (10) de forme sphérique destinée à coopérer avec une surface de glissement concave de même forme sphérique et de même diamètre d'une pièce de réception de la tête, dans laquelle le corps de ladite pièce et celui de ladite tête (1) sont métalliques, et caractérisée d'autre part en ce qu'elle comprend un piège à particules sous la forme d'une fossette ménagée dans ladite surface de glissement concave de ladite pièce de réception de la tête.

2. Partie articulaire d'une prothèse de hanche selon la revendication 1, caractérisée en ce que ladite fossette formant piège à particules contient le pôle (P) de ladite surface de glissement concave de ladite pièce de réception de la tête.

3. Partie articulaire d'une prothèse de hanche selon la revendication 1 ou 2, caractérisée en ce que ladite pièce de réception de la tête est une cupule mobile (3) présentant une surface de glissement externe (31) destinée à coopérer avec la surface de glissement naturelle d'une cavité cotyloïdienne.

4. Partie articulaire d'une prothèse de hanche selon la revendication 1 ou 2, caractérisé en ce que ladite pièce de réception de la tête est un cotyle prothétique (4).

5. Partie articulaire d'une prothèse de hanche selon la revendication 4, elle-même dépendante de la revendication 2, caractérisée en ce que ladite fossette (42) est ouverte sur la face externe du cotyle (4) par un alésage polaire taraudé (43).

6. Partie articulaire d'une prothèse de hanche selon la revendication 1 ou 2, caractérisée en ce que ladite pièce de réception de la tête est un élément intermédiaire mobile (5) entre la tête (1) et un cotyle prothétique (4), en ce que le corps du cotyle (4) est métallique, et en ce que ce dernier comporte lui aussi un piège à particules (42).

7. Partie articulaire d'une prothèse de hanche selon l'une des revendications 1 à 6, caractérisée en ce que ladite tête présente une fossette polaire (11) dans sa surface de glissement, ou bien un aplatissement ou méplat polaire.

8. Partie articulaire d'une prothèse de hanche selon l'une des revendications 1 à 7, caractérisée en ce qu'au moins l'une de ses surfaces de glissement (10, 30, 31, 40, 50, 51) est en métal dur hyperlisse.

9. Partie articulaire d'une prothèse de hanche selon l'une des revendications 1 à 7, caractérisée en ce qu'au moins l'une de ses surfaces de glissement (10, 30, 31, 40, 50, 51) est un revêtement en céramique réalisé par la technique de dépôt sous vide.

10. Partie articulaire d'une prothèse de hanche selon l'une des revendications 1 à 7, caractérisée en ce qu'au moins l'une de ses surfaces de glissement (10, 30, 31, 40, 50, 51) est un revêtement en céramique réalisé par la technique dite "de la céramique projetée".

11. Partie articulaire d'une prothèse de hanche selon la revendication 10, caractérisée en ce que ses surfaces de glissement consistant en un revêtement en céramique réalisé par la technique dite de "la céramique projetée" sont interrompues par une partie polaire en creux, ou un aplatissement polaire pour ce qui concerne ladite tête (1).
